Europäisches Patentamt

European Patent Office

Office européen des brevets

(11) Publication number: **0 464 553 A2**

# EUROPEAN PATENT APPLICATION

(21) Application number: **91110368.7**

(22) Date of filing: **24.06.91**

(51) Int. Cl.5: **C12N 15/12**, C07K 15/00, C12P 21/02

(30) Priority: **04.07.90 JP 175174/90**

(43) Date of publication of application:
**08.01.92 Bulletin 92/02**

(84) Designated Contracting States:
**AT BE CH DE DK ES FR GB GR IT LI LU NL SE**

(71) Applicant: **SANWA KAGAKU KENKYUSHO CO., LTD.**
**No. 35, Higashi-sotobori-cho**
**Higashi-ku Nagoya-shi Aichi-ken(JP)**

(72) Inventor: **Natori, Shunji**
**2208-19, Fukawa, Tone-machi**
**Kita-soma-gun, Ibaragi-ken(JP)**
Inventor: **Jomori, Takahito, Sanwa Kagaku**
**Kenkyusho Co. Ltd.**
**35, Higashi-sotobori-cho, Higashi-ku, Nagoya**
**Aichi-ken(JP)**
Inventor: **Sawai, Kiichi, c/o Sanwa Kagaku**
**Kenkyusho Co. Ltd.**
**35, Higashi-sotobori-cho, Higashi-ku, Nagoya**
**Aichi-ken(JP)**

(74) Representative: **Wächtershäuser, Günter, Dr.**
**Tal 29**
**W-8000 München 2(DE)**

(54) **DNA clone encoding precursor of lectin-like substance from Periplaneta americana and cloning therefor.**

(57) There are disclosed a DNA clone encoding a precursor of lectin-like substance with binding ability to lipopolysaccharides and from Periplaneta americana, a process for the preparation of the clone and a recombinant plasmid with an insert of the clone region. The lectin-like substance can be used for the separation of bacteria by utilizing its characteristic of high specificity to lipopolysaccharides.

EP 0 464 553 A2

The present invention relates to a DNA clone encoding a precursor of lectin-like substance with binding ability to lipopolysaccharides and from a cockroach and more particularly Periplaneta americana, a process for the preparation of the DNA clone, and a plasmid wherein the DNA is inserted.

Lectin is a sugurbinding protein found in animals, plants and bacteria inclusive of virus, excepting those of products due to immunoreaction. The lectins obtained from plants had been known for a long time as a hemagglutinin.

Recently, various lectin-like substances have been found not only in the plants, but also in animals, and their physical significance and characteristics have occupied the attention in the pharmacological fields, since some of them shows an agglutination specificity not only to erythrocyte, but also leukocyte, cancer cell and the like [Jap. Pat. No. Sho 63 - 209530(A)]. However, there are no many reports on the lectin-like substance with high specificity, and almost no report has been issued on the substance which show high specificity to a lipopolysaccharide presenting in an outer surface of bacteria.

The lectin-like substance with binding ability to lipopolysaccharides, to which the present invention is concerned, was isolated for the first time by the present inventors themselves from a body fluid of Periplaneta americana [Jap. Pat. No. Hei 2 - 88599(A)].

This known lectin-like substance has an average molecular weight of about 450,000 and is constituted with subunits, each having molecular weight of about 28000. Characteristics of the substance lies in specificity to D-fucose, L-fucose, D-glucose, D-galactose and the like monosaccharides, and in showing a strong affinity to a chain of lipopolysaccharide in Escherichia coli.

Therefore, the substance can be used in various purposes, for instance a removal of the lipopolysaccharide contaminated in a process for the production of medicines, separation of bacteria, a reagent of quantitative measurement of the lipopolysaccharide, or the like.

According to the technique as disclosed in said Jap. Pat. No. Hei 2 - 88599(A), the lectin-like substance with binding ability to lipopolysaccharides has been obtained from the body fluid of Periplaneta americana using extraction and purification procedures and thus it was quite difficult to obtain them in a large amount and with a reasonable cost.

A basic matter of the invention lies in developing an industrially acceptable process for the preparation of a lectin-like substance with binding ability to lipopolysaccharides and its related substrances, by utilizing techniques so called "Bio-Technologies".

A principal object of the invention is to provide a DNA clone which encodes a precursor of such lectin-like substance.

An additional object of the invention is to provide a process for the preparation of the DNA clone.

Another object of the invention is to provide a plasmid containing the DNA clone region or its fragment, for instance a region encoding the lectin-like substance per se is inserted, so that a microorganism or animal cell transformed by the plasmid expresses the substance to allow a large scale production thereof.

The inventors have carefully studied and investigated to finally obtain the DNA clone for the precursor of lectin-like substance in question, therefor such a basic matter or problem previously described has been dissolved to establish the invention.

According to the invention, the principal object is attained by a single-stranded DNA clone with the nucleotide sequence of

```
5'--- ATG ATG AAT ACG AGA GCC TTA CTG CCC CTG TCA GTA CTA
      CTC ATG GCT ACC TTG TGT CTG TGT GAG CTG CCT ATC CCC
      ATA CTG CAA AGG TTC GTG CGT TCA GTT CCT GAG GAG TGT
      CCT ATA GCT GAC CCG AGT GAC TTC AAG TTT TCG ATC ACG
      AGC AAT AGG AAC AAG ACT GGC CAC TGG ACG GCG CAG GTG
      CGA CTG GAA CAC GGG GAA CAT GAA CAG TCA GGC TCT AAC
      CAA CAT AAC AGA GAT CTC TGG CAA GTG GAT CTG GAG CAG
      ACC ACA ACC ACT TGT GCA GGC GTC AAG TCT GTT CAG ATC
      ATT ACT ACA ATC ACA GCA CCT CCT CCT ACT GCC GCT CCC
      AGT ATT CCT CCA GGA TAC GAG TTG TCC GCT GTT CTG GGA
      TAT TAC AAA TTT CAT AAA ACT CCG AAG ACT TGG GAT GAA
      GCA AGA ATA ATC TGT CAA CAG GAG GGA GGA CAT CTG GTC
      ATA ATA AAT TCG GAG GAC GAA TCT AAA GTT CTT CAA AAC
      CTT TTC AGC AAA GTG ACG AAG ACG GAA GGA GCA ACA AAC
      AAC GAC TAC ATA TTT ATC GGC ATT CAT GAT AGA TTT GTT
      GAA GCA GAA TTC ATC ACG ATA TTT GGA AAA CCT CTG GCT
      ACT ACT GGA TTC ACT CGT TGG GTG GAC TCT ATC AAC CCT
      GAC AAT GCT GGA GGT AAC GAG AAC TGT GGC TCC ATG CAT
      CCT AAT GGC GGT TTG AAC GAT ATA CCA TGC CCG TGG AAA
      CTG CCC TTC GTC TGT GAA GTG GAG CTC
```

wherein A, C, G and T are, respectively, a deoxyribonucleotide having adenine, cytosine, guanine or thymine base and the sequence is given as that of each codon encoding a specified amino acid, or a nucleotide sequence containing a part of the sequence.

The single-stranded DNA clone according to the invention, therefore, encodes amino acids with the sequence of

Met-Met-Asn-Thr-Arg-Ala-Leu-Leu-Pro-Leu-Ser-Val-Leu-
Leu-Met-Ala-Thr-Leu-Cys-Leu-Cys-Glu-Leu-Pro-IIe-Pro-
Ile-Leu-Gln-Arg-Phe-Val-Arg-Ser-Val-Pro-Glu-Glu-Cys-
Pro-Ile-Ala-Asp-Pro-Ser-Asp-Phe-Lys-Phe-Ser-1le-Thr-
Ser-Asn-Arg-Asn-Lys-Thr-Gly-His-Trp-Thr-Ala-Gln-Val-
Arg-Leu-Glu-His-Gly-Glu-His-Glu-Gln-Ser-Gly-Ser-Asn-
Gln-His-Asn-Arg-Asp-Leu-Trp-Gln-Val-Asp-Leu-Glu-Gln-
Thr-Thr-Thr-Thr-Cys-Ala-Gly-Val-Lys-Ser-Val-Gln-1le-
1le-Thr-Thr-1le-Thr-Ala-Pro-Pro-Pro-Thr-Ala-Ala-Pro-
Ser-1le-Pro-Pro-Gly-Tyr-Glu-Leu-Ser-Ala-Val-Leu-Gly-
Tyr-Tyr-Lys-Phe-His-Lys-Thr-Pro-Lys-Thr-Trp-Asp-Glu-
Ala-Arg-1le-1le-Cys-Gln-Gln-Glu-Gly-Gly-His-Leu-Val-
1le-1le-Asn-Ser-Glu-Asp-Glu-Ser-Lys-Val-Leu-Gln-Asn-
Leu-Phe-Ser-Lys-Val-Thr-Lys-Thr-Glu-Gly-Ala-Thr-Asn-
Asn-Asp-Tyr-1le-Phe-1le-Gly-Ile-His-Asp-Arg-Phe-Val-
Glu-Gly-Glu-Phe-Ile-Thr-Ile-Phe-Gly-Lys-Pro-Leu-Ala-
Thr-Thr-Gly-Phe-Thr-Arg-Trp-Val-Asp-Ser-1le-Gln-Pro-
Asp-Asn-Ala-Gly-GIy-Asn-Glu-Asn-Cys-Gly-Ser-Met-His-
Pro-Asn-Gly-Gly-Leu-Asn-Asp-Ile-Pro-Cys-Pro-Trp-Lys-
Leu-Pro-Phe-Val-Cys-Glu-Val-Glu-Leu.

According to the amino acid sequence from DNA of precursor for the lectin-like substance, the lectin-like substance is encoded in the region of 34th to 256th amino acid residues counting from the methionine residue (Met) designated by the initiation codon or triplet of ATG. It seems to be that the region consisting of 33 amino acids from Met to Arg corresponds to a signal peptide relating to a secretion of the lectin-like substance, in view of its structure.

According to a process of the invention, the double-stranded DNA clone which encodes a region containing the lectin-like substance with binding ability to lipopolysaccharides can be prepared by converting mRNA from fat-body of Periplaneta americana into cDNA, constructing a cDNA library with use of the cDNA and vector/primer DNA to carry out a transformation of Escherichia coli. Probes for cDNA clone were synthesizing, which are a mixture of 24 tetracosamers, each consisting of 20 deoxyribonucleotides of the formula

$$5' \text{ --- GGA} \big\} \text{ TC1GCA} \big\} \text{ ATIGGA} \big\} \text{ CAT} \big\} \text{ TC}$$
$$\text{G} \quad \text{G} \quad \text{G} \quad \text{C} \qquad (A)$$
$$\text{T}$$

wherein I is inosine residue and was inserted in each position where anyone of A, C G and T may occupy,
which is complementary to mRNA corresponding to
    Glu-Cys-Pro-Ile-Ala-Asp-Pro
of 5th to 11th amino acids from 5'-terminal in an amino acid sequence for lectin-like substance from a body fluid of Periplaneta americana, and labeled at 5'-terminal of the synthesized oligodeoxyribonucleotides. Transformants derived from a cDNA library were screened through a hybridization using the labeled oligodeoxyribonucleotides as probes to obtain a positive clone which hybridize with those probes.

The grounds that the oligodeoxyribonucleotides corresponding to 5th to 11th amino acids for the sequence of the lectin-like substance were selected as the probes and that the inosine was inserted in all of positions where A, C, G or T may occupy lie in making minimum the kinds of oligodeoxyribonucleotides responding to the the amino acid sequence (A) to save the time for synthesizing it.

A single-stranded DNA clone can be obtained by separating the resulting double-stranded DNA clone with use of a known method, for instance treating the same at 90°C for about 3 minutes and then cooling with an ice bath.

The single or double stranded DNA clone can be made into a fragment in various length, in accordance with conventional techniques, for instance by treating with a suitable restriction enzyme(s), binding or ligating fragments, synthesizing a region which can not be obtained through a cleaving technique, binding the synthesized region to the fragment obtained through the enzymatical treatment to obtain a desired DNA fragment such as that consisting of only the region encoding the lectin-like substance with binding ability to lipopolysaccharides.

The double-stranded DNA clone comprising the region encoding the precursor of lectin-like substance or the fragment thereof can be inserted into a plasmid, in accordance with a known method, for instance taking out a plasmid from Escherichia coli, purifying the plasmid, cleaving the plasmid with a restriction enzyme at specified nucleotide position, ligating with a DNA ligase the double-stranded DNA to each site of the cleavages of the cleaved plasmid to re-construct the plasmid into recombinant one.

If the recombinant plasmid is inserted into Escherichia coli or an animal cell to transform the same and the resulting transformant is cultured, the transformant shall express the lectin-like substance in accordance with its gene information, so that the substance will be obtained by carrying out convention isolation and purification procedures.

The invention will now be further explained in more detail with Examples and Test Example which shall refer to drawings, wherein

Fig. 1 shows a cDNA clone region including that encoding a precursor of lectin-like substance with binding ability to lipopolysaccharides and from Periplaneta americana, and a strategy for sequencing a nucleotide sequence in the region together with a map of restriction enzymes for the sequencing;

Fig. 2 shows a determined nucleotide sequence of the cDNA clone as well as an amino acid sequence translated from the nucleotide sequence; and

Fig. 3 illustrates steps for preparing a recombinant plasmid, wherein a fragment comprising a signal region and the precursor region is inserted.

Example 1

(a) Preparation of poly(A)RNA comprising mRNA of lectin-like substance with binding ability to lipopolysaccharides

In accordance with the method as disclosed by Chirgwin J. M. et al ["Biochemistry", Vol. 18, pages 5294 - 5299 (1979)], the total RNA (0.9mg) was extracted from a raw material of fat-body of Periplaneta americana with use of 4M-guanidium thiocyanate bound to an oligo(dT)cellulose column with use of 10mM-Tris hydrochloride buffer containing 0.5M-KCl, and then eluted with use of the buffer but free from KCl to obtain poly(A)RNA (about 56µ g).

(b) Synthesis of DNA complementary to mRNA

In the first place, a mixture of 24 tetracosamers, each consisting of 20 deoxyribonucleotides of the formula

$$5' \ \text{---} \ \text{GGA}\!\!\left.\begin{array}{c}\\G\end{array}\right\} \text{TCIGCA}\!\!\left.\begin{array}{c}\\G\\T\end{array}\right\} \text{ATIGGA}\!\!\left.\begin{array}{c}\\G\end{array}\right\} \text{CAT}\!\!\left.\begin{array}{c}\\C\end{array}\right\} \text{TC} \qquad (A)$$

wherein I is inosine residue and was inserted in each position where anyone of A, C G and T may occupy,
which is complementary to mRNA corresponding to
   Glu-Cys-Pro-Ile-Ala-Asp-Pro
of 5th to 11th amino acids from 5'-terminal in an amino acid sequence for lectin-like substance and from a body fluid of Periplaneta americana was prepared with use of a DNA synthesizer (Model 381A, marketed from Applied Biosystems Co.).

(c) Construction of cDNA library

A cDNA library was constructed with use of 35$\mu$ g of the poly(A)RNA and 4.3$\mu$ g of a vector/primer DNA, in accordance with the method as described by Okayama, H. and Berg, P. ["Mol. Cell. Bio.", Vol. 2, pages 161 - 170 (1982)], and Escherichia coli (HB 101 strain) was transformed in accordance with the method as described by Morrison, D. A. ["Methods in Enzymol.", Vol. 68, pages 326 - 331 (1979)]. Through a screening with use of LB-agar plate containing 50$\mu$ g/ml of ampicillin, ampicillin resistant transformants were obtained by about 9000 cells per 1$\mu$ g of poly(A)RNA, namely about 320000 cells in total.

(d) cDNA cloning

The cloning was carried out in accordance with the method as described by Hanahan, D. et al ["Gene", Vol. 10, pages 63 - 67 (1980)].

Namely, about 60000 cells among those about 320000 ampicillin resistant transformants as described in Item (c) were replicated on a nitrocellulose filter, cultured for 3 to 4 hours on an agar plate containing 50$\mu$ g/ml of ampicillin, and then transferred on LB-agar plate containing 500$\mu$ g/ml of chloramphenicol to further culture the same at 37$^\circ$C for one overnight.

A colony formed on the filter was subjected to bacteriolysis, namely a double-stranded DNA was made into a single-stranded DNA with use of 0.5N-NaOH, fixed on a filter, neutralized to make pH to 7.5, and dipped the filter for 5 minutes in Tris-hydrochloride buffer (pH 7.5) containing 1.5M-NaCl to remove bacterial fragments and the like other than DNA. Thereafter, the filter was air dried and baked for 2 hours at 80$^\circ$C to obtain a testing filter for screening.

While, the screening of the transformants was carried out as follows, in accordance with the method as described by Grunstein, M. et al ["Proc. Natl. Acad. Sci. U.S.A.", Vol. 72, pages 3961 - 3965 (1975)].

Each of the oligodeoxyribonucleotides synthesized by the method as described in Item (b) and shown by said Formula (A) was end-labeled at 5'-terminal with [Y - $^{32}$P]ATP (5000 Ci/mmol) and T4 polynucleotide kinase to make the oligodeoxyribonucleotides into probes for screening. A relative activity of each probes was 1 to 2 x 10$^6$ cpm/pmol.

The transformants on the testing filter were screened by a hybridization at 50$^\circ$C with use of the probes and judged by the autoradiogram method to find that only 4 transformants among about 60000 transformants are positive clones.

As these positive clones gave identical restriction enzyme map, only one of these cloned DNAs was analyzed as stated in the Test Example given later. This clone has a cDNA insert region of about 2300bp including poly(dA) (dT) tails and poly(dG) (dC) tracts.

Test Example

(Determination of nucleotide and amino acid sequences)

A nucleotide sequence of the cDNA was determined in accordance with the dideoxy method as described by Hattori et al ["Anal. Biochem.", Vol. 152, pages 232 - 238 (1986)] in which various deletion fragments for sequencing obtained by cleaving the DNA clone with exonuclease II and mung bean nuclease.

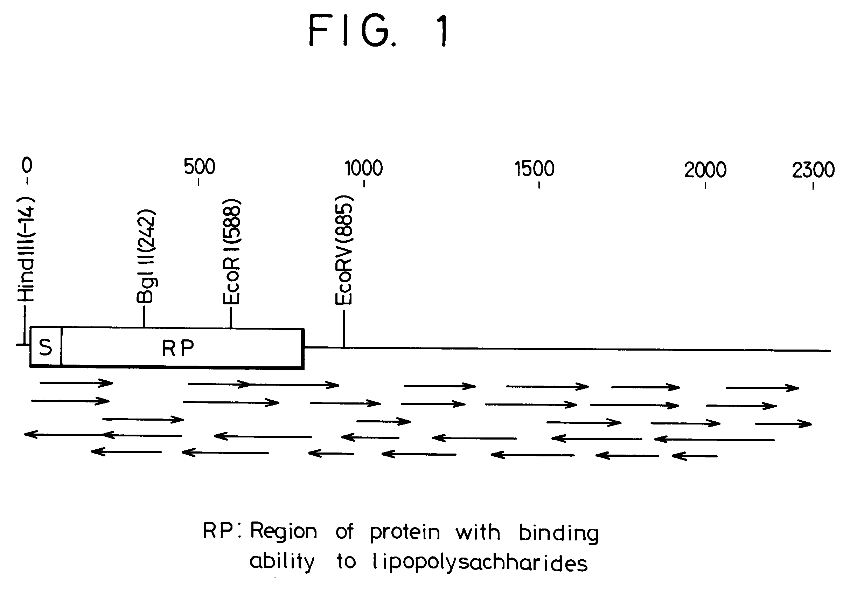

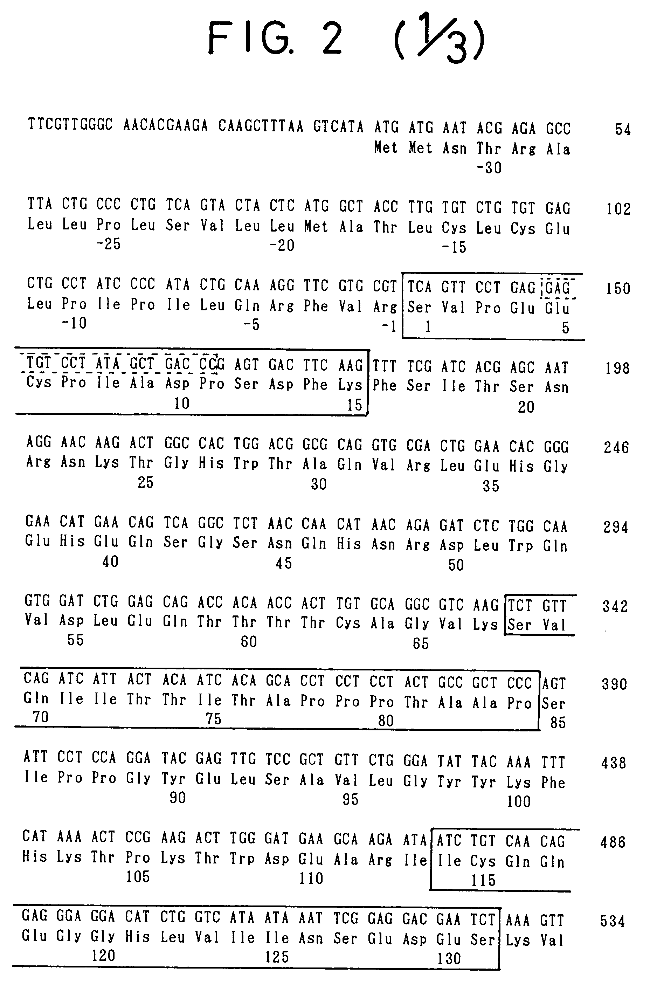

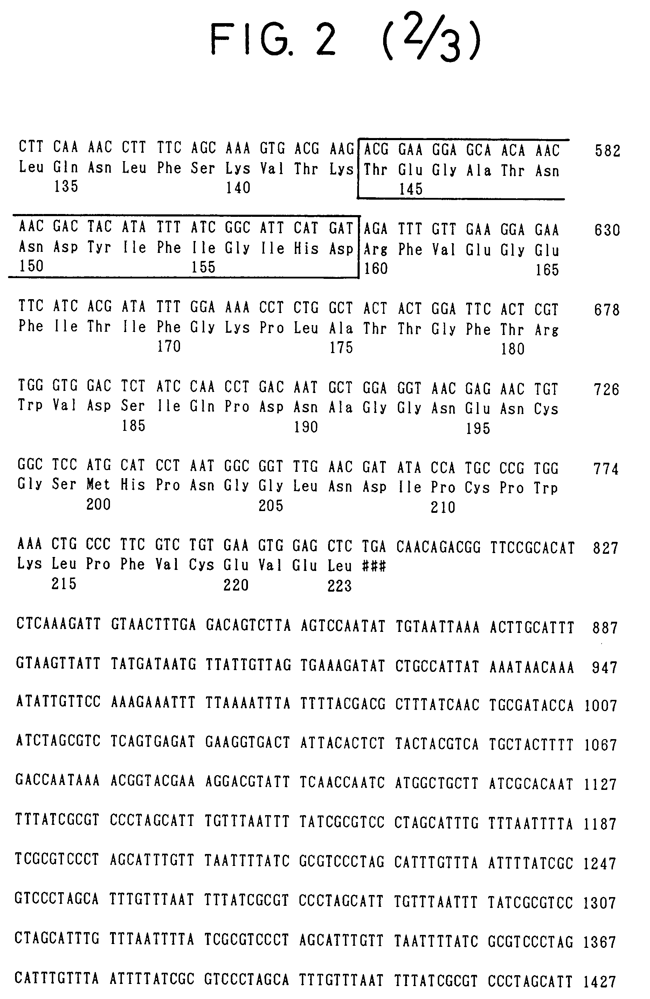

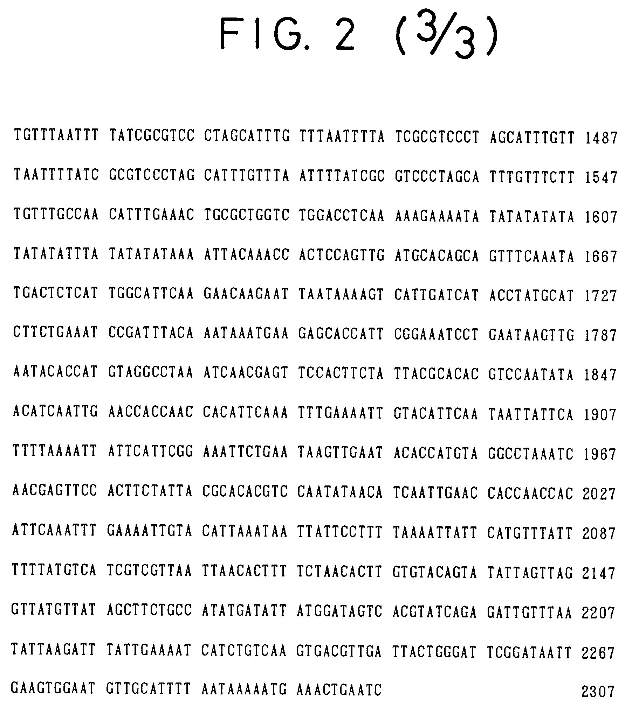

In Fig. 1, there is shown a cDNA clone region including that encoding a precursor of lectin-like substance with binding ability to lipopolysaccharides, and a strategy for sequencing a nucleotide sequence in the region together with a map of restriction enzymes. The numerals at uppermost portion in the Figure are nucleotide number. The "HindIII" and the like are names of restriction enzymes and a numeral given in parentheses shows a cleavage portion due to the restriction enzyme and given under assumption of that the adenine residue at 37th position in Fig. 2 is the first nucleotide. The boxed region is the precursor and consists of a putative signal region (S) and a region (RP) to be estimated as corresponding to the lectin-like substance with binding ability to lipopolysaccharides. In the lower portion in Figure 1, each of the horizontal arrow shows a direction and limit of nucleotide sequencing.

In Fig. 2, there are shown thus determined nucleotide sequence of cDNA insert region encoding a region containing the precursor region as well as an amino acid sequence which can be translated from the nucleotide sequence. In Fig. 2, the numeral given at left side is the number of nucleotides and another numeral given at lower side of the amino acid sequence is number of amino acid residues. Regions boxed with a solid line show regions with an amino acid sequence same with that for a fragment digested by trypsin on the lectin-like substance from a body fluid of Periplaneta americana, namely the substance disclosed in Jap. Pat. No. Hei 2 - 88599(A) which is introduced in preamble part of this specification. A region boxed with doted line shows a region selected as probes for screening the transformants in the Example.

The precursor of lectin-like substance lies as a long open reading frame beginning from an initiation codon of ATG and ending at termination codon of TGA. From the Figure, it seems to be that the initial portion of precursor, namely -33th Met to -1st Arg constitutes a signal sequence (S region in Fig. 1) which generally be found on secretory proteins, in view of its structure consisting of relative hydrophobic amino acids, and more particularly from -28th Ala to -1st Arg.

Example 2

(Preparation of recombinant plasmid)

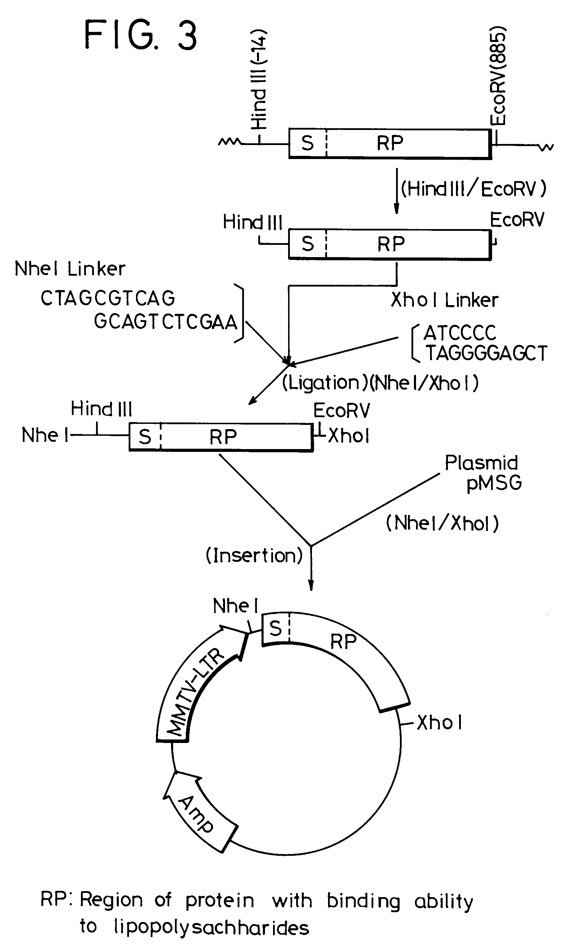

This embodiment will be explained with reference to the drawings and more particularly to Fig. 3.

In the first place, the DNA clone (double-stranded DNA) was cleaved with restriction enzymes of HindIII and EcoRV to obtain a fragment of about 900bp. On the other hand, NHeI and XhoI linkers were prepared with use of a DNA synthesizer (marketed by Applied Biosystems Co.) and were ligated to HindIII and EcoRV cleavage sites of the fragment, respectively.

While, a commercially available plasmid ("pMSG" marketed by Pharmacia S.p.A.) for expression with an animal cell was cleaved with use of restriction enzymes of NheI and XhoI. To the resulting cohesive ends, these fragments with the linkers were ligated to reconstruct the plasmid.

As shown in the last portion in Fig. 3, the resulting plasmid has the DNA clone insert region of the precursor at downstream of a powerful promoter of a long terminal repeat on mouse mammary tumor virus (MMTV-LTR). If L or CHO cells are transformed with use of such recombinant plasmid and cultured, therefore, the cells will express the precursor.

7

SEQUENCE LISTING

SEQ ID NO : 1

SEQUENCE TYPE : Nucleotide with corresponding protein

SEQUENCE LENGTH : 2307 base pairs


STRANDNESS : single

TOPOLOGY : linear

MOLECULE TYPE : cDNA to mRNA


ORIGINAL SOURCE

ORGANISM : cockroach (<u>Periplaneta</u> <u>americana</u>)


FEATURES :

from 37 to 135 bp signal peptide

from 136 to 804 bp peptide as precursor of lectin-like substance with binding ability to liposaccharides


```
TTCGTTGGGC AACACGAAGA CAAGCTTTAA GTCATA ATG ATG AAT ACG AGA GCC      54
                                        Met Met Asn Thr Arg Ala
                                                          -30

TTA CTG CCC CTG TCA GTA CTA CTC ATG GCT ACC TTG TGT CTG TGT GAG     102
Leu Leu Pro Leu Ser Val Leu Leu Met Ala Thr Leu Cys Leu Cys Glu
        -25                 -20                 -15

CTG CCT ATC CCC ATA CTG CAA AGG TTC GTG CGT TCA GTT CCT GAG GAG     150
Leu Pro Ile Pro Ile Leu Gln Arg Phe Val Arg Ser Val Pro Glu Glu
        -10                 -5          -1   1               5

TGT CCT ATA GCT GAC CCG AGT GAC TTC AAG TTT TCG ATC ACG AGC AAT     198
Cys Pro Ile Ala Asp Pro Ser Asp Phe Lys Phe Ser Ile Thr Ser Asn
                    10              15              20

AGG AAC AAG ACT GGC CAC TGG ACG GCG CAG GTG CGA CTG GAA CAC GGG     246
Arg Asn Lys Thr Gly His Trp Thr Ala Gln Val Arg Leu Glu His Gly
            25              30              35
```

8

```
GAA CAT GAA CAG TCA GGC TCT AAC CAA CAT AAC AGA GAT CTC TGG CAA     294
Glu His Glu Gln Ser Gly Ser Asn Gln His Asn Arg Asp Leu Trp Gln
        40                      45                  50

GTG GAT CTG GAG CAG ACC ACA ACC ACT TGT GCA GGC GTC AAG TCT GTT     342
Val Asp Leu Glu Gln Thr Thr Thr Thr Cys Ala Gly Val Lys Ser Val
        55                  60                  65

CAG ATC ATT ACT ACA ATC ACA GCA CCT CCT CCT ACT GCC GCT CCC AGT     390
Gln Ile Ile Thr Thr Ile Thr Ala Pro Pro Pro Thr Ala Ala Pro Ser
    70                  75                  80                  85

ATT CCT CCA GGA TAC GAG TTG TCC GCT GTT CTG GGA TAT TAC AAA TTT     438
Ile Pro Pro Gly Tyr Glu Leu Ser Ala Val Leu Gly Tyr Tyr Lys Phe
                    90                  95                  100

CAT AAA ACT CCG AAG ACT TGG GAT GAA GCA AGA ATA ATC TGT CAA CAG     486
His Lys Thr Pro Lys Thr Trp Asp Glu Ala Arg Ile Ile Cys Gln Gln
            105                 110                 115

GAG GGA GGA CAT CTG GTC ATA ATA AAT TCG GAG GAC GAA TCT AAA GTT     534
Glu Gly Gly His Leu Val Ile Ile Asn Ser Glu Asp Glu Ser Lys Val
        120                 125                 130

CTT CAA AAC CTT TTC AGC AAA GTG ACG AAG ACG GAA GGA GCA ACA AAC     582
Leu Gln Asn Leu Phe Ser Lys Val Thr Lys Thr Glu Gly Ala Thr Asn
        135                 140                 145

AAC GAC TAC ATA TTT ATC GGC ATT CAT GAT AGA TTT GTT GAA GGA GAA     630
Asn Asp Tyr Ile Phe Ile Gly Ile His Asp Arg Phe Val Glu Gly Glu
150                 155                 160                 165

TTC ATC ACG ATA TTT GGA AAA CCT CTG GCT ACT ACT GGA TTC ACT CGT     678
Phe Ile Thr Ile Phe Gly Lys Pro Leu Ala Thr Thr Gly Phe Thr Arg
                    170                 175                 180

TGG GTG GAC TCT ATC CAA CCT GAC AAT GCT GGA GGT AAC GAG AAC TGT     726
Trp Val Asp Ser Ile Gln Pro Asp Asn Ala Gly Gly Asn Glu Asn Cys
            185                 190                 195

GGC TCC ATG CAT CCT AAT GGC GGT TTG AAC GAT ATA CCA TGC CCG TGG     774
Gly Ser Met His Pro Asn Gly Gly Leu Asn Asp Ile Pro Cys Pro Trp
        200                 205                 210

AAA CTG CCC TTC GTC TGT GAA GTG GAG CTC TGA CAACAGACGG TTCCGCACAT   827
Lys Leu Pro Phe Val Cys Glu Val Glu Leu ###
        215                 220         223

CTCAAAGATT GTAACTTTGA GACAGTCTTA AGTCCAATAT TGTAATTAAA ACTTGCATTT   887
```

9

```
GTAAGTTATT TATGATAATG TTATTGTTAG TGAAAGATAT CTGCCATTAT AAATAACAAA  947

ATATTGTTCC AAAGAAATTT TTAAAATTTA TTTTACGACG CTTTATCAAC TGCGATACCA 1007

ATCTAGCGTC TCAGTGAGAT GAAGGTGACT ATTACACTCT TACTACGTCA TGCTACTTTT 1067

GACCAATAAA ACGGTACGAA AGGACGTATT TCAACCAATC ATGGCTGCTT ATCGCACAAT 1127

TTTATCGCGT CCCTAGCATT TGTTTAATTT TATCGCGTCC CTAGCATTTG TTTAATTTTA 1187

TCGCGTCCCT AGCATTTGTT TAATTTTATC GCGTCCCTAG CATTTGTTTA ATTTTATCGC 1247

GTCCCTAGCA TTTGTTTAAT TTTATCGCGT CCCTAGCATT TGTTTAATTT TATCGCGTCC 1307

CTAGCATTTG TTTAATTTTA TCGCGTCCCT AGCATTTGTT TAATTTTATC GCGTCCCTAG 1367

CATTTGTTTA ATTTTATCGC GTCCCTAGCA TTTGTTTAAT TTTATCGCGT CCCTAGCATT 1427

TGTTTAATTT TATCGCGTCC CTAGCATTTG TTTAATTTTA TCGCGTCCCT AGCATTTGTT 1487

TAATTTTATC GCGTCCCTAG CATTTGTTTA ATTTTATCGC GTCCCTAGCA TTTGTTTCTT 1547

TGTTTGCCAA CATTTGAAAC TGCGCTGGTC TGGACCTCAA AAAGAAAATA TATATATATA 1607

TATATATTTA TATATATAAA ATTACAAACC ACTCCAGTTG ATGCACAGCA GTTTCAAATA 1667

TGACTCTCAT TGGCATTCAA GAACAAGAAT TAATAAAAGT CATTGATCAT ACCTATGCAT 1727

CTTCTGAAAT CCGATTTACA AATAAATGAA GAGCACCATT CGGAAATCCT GAATAAGTTG 1787

AATACACCAT GTAGGCCTAA ATCAACGAGT TCCACTTCTA TTACGCACAC GTCCAATATA 1847

ACATCAATTG AACCACCAAC CACATTCAAA TTTGAAAATT GTACATTCAA TAATTATTCA 1907

TTTTAAAATT ATTCATTCGG AAATTCTGAA TAAGTTGAAT ACACCATGTA GGCCTAAATC 1967

AACGAGTTCC ACTTCTATTA CGCACACGTC CAATATAACA TCAATTGAAC CACCAACCAC 2027

ATTCAAATTT GAAAATTGTA CATTAAATAA TTATTCCTTT TAAAATTATT CATGTTTATT 2087

TTTTATGTCA TCGTCGTTAA TTAACACTTT TCTAACACTT GTGTACAGTA TATTAGTTAG 2147

GTTATGTTAT AGCTTCTGCC ATATGATATT ATGGATAGTC ACGTATCAGA GATTGTTTAA 2207

TATTAAGATT TATTGAAAAT CATCTGTCAA GTGACGTTGA TTACTGGGAT TCGGATAATT 2267

GAAGTGGAAT GTTGCATTTT AATAAAAATG AAACTGAATC                       2307
```

10

**Claims**

1.  A single-stranded DNA clone encoding an amino acid sequence for a precursor of lectin-like substance with binding ability to lipopolysaccharides and from Periplaneta americana, which comprises nucleotides with the sequence of

```
5'--- ATG ATG AAT ACG AGA GCC TTA CTG CCC CTG TCA GTA CTA
      CTC ATG GCT ACC TTG TGT CTG TGT GAG CTG CCT ATC CCC
      ATA CTG CAA AGG TTC GTG CGT TCA GTT CCT GAG GAG TGT
      CCT ATA GCT GAC CCG AGT GAC TTC AAG TTT TCG ATC ACG
      AGC AAT AGG AAC AAG ACT GGC CAC TGG ACG GCG CAG GTG
      CGA CTG GAA CAC GGG GAA CAT GAA CAG TCA GGC TCT AAC
      CAA CAT AAC AGA GAT CTC TGG CAA GTG GAT CTG GAG CAG
      ACC ACA ACC ACT TGT GCA GGC GTC AAG TCT GTT CAG ATC
      ATT ACT ACA ATC ACA GCA CCT CCT CCT ACT GCC GCT CCC
      AGT ATT CCT CCA GGA TAC GAG TTG TCC GCT GTT CTG GGA
      TAT TAC AAA TTT CAT AAA ACT CCG AAG ACT TGG GAT GAA
      GCA AGA ATA ATC TGT CAA CAG GAG GGA GGA CAT CTG GTC
      ATA ATA AAT TCG GAG GAC GAA TCT AAA GTT CTT CAA AAC
      CTT TTC AGC AAA GTG ACG AAG ACG GAA GGA GCA ACA AAC
      AAC GAC TAC ATA TTT ATC GGC ATT CAT GAT AGA TTT GTT
      GAA GCA GAA TTC ATC ACG ATA TTT GGA AAA CCT CTG GCT
      ACT ACT GGA TTC ACT CGT TGG GTG GAC TCT ATC CAA CCT
      GAC AAT GCT GGA GGT AAC GAG AAC TGT GGC TCC ATG CAT
      CCT AAT GGC GGT TTG AAC GAT ATA CCA TGC CCG TGG AAA
      CTG CCC TTC GTC TGT GAA GTG GAG CTC
```

wherein A, C, G and T are, respectively, a deoxyribonucleotide having adenine, cytosine, guanine or thymine base and said sequence is given as that of each codon encoding a specified amino acid, or a nucleotide sequence with a degeneracy to the sequence.

2.  A single-stranded DNA as claimed in Claim 1, wherein these nucleotides encodes amino acids with the sequence of

```
Met-Met-Asn-Thr-Arg-Ala-Leu-Leu-Pro-Leu-Ser-Val-Leu-
Leu-Met-Ala-Thr-Leu-Cys-Leu-Cys-Glu-Leu-Pro-Ile-Pro-
Ile-Leu-Gln-Arg-Phe-Val-Arg-Ser-Val-Pro-Glu-Glu-Cys-
Pro-lle-Ala-Asp-Pro-Ser-Asp-Phe-Lys-Phe-Ser-lle-Thr-
Ser-Asn-Arg-Asn-Lys-Thr-Gly-His-Trp-Thr-Ala-Gln-Val-
Arg-Leu-Glu-His-Gly-Glu-His-Glu-Gln-Ser-Gly-Ser-Asn-
Gln-His-Asn-Arg-Asp-Leu-Trp-Gln-Val-Asp-Leu-Glu-Gln-
Thr-Thr-Thr-Thr-Cys-Ala-Gly-Val-Lys-Ser-Val-Gln-Ile-
IIe-Thr-Thr-IIe-Thr-Ala-Pro-Pro-Pro-Thr-Ala-Ala-Pro-
Ser-Ile-Pro-Pro-Gly-Tyr-Glu-Leu-Ser-Ala-Val-Leu-Gly-
Tyr-Tyr-Lys-Phe-His-Lys-Thr-Pro-Lys-Thr-Trp-Asp-Glu-
Ala-Arg-lle-lIe-Cys-Gln-Gln-Glu-Gly-Gly-His-Leu-Val-
lle-Ile-Asn-Ser-Glu-Asp-Glu-Ser-Lys-Val-Leu-Gln-Asn-
Leu-Phe-Ser-Lys-Val-Thr-Lys-Thr-GIu-GIy-Ala-Thr-Asn-
Asn-Asp-Tyr-lle-Phe-lle-Gly-IIe-His-Asp-Arg-Phe-Val-
Glu-Gly-Glu-Phe-IIe-Thr-Ile-Phe-Gly-Lys-Pro-Leu-Ala-
Thr-Thr-GIy-Phe-Thr-Arg-Trp-Val-Asp-Ser-Ile-Gln-Pro-
Asp-Asn-Ala-Gly-GIy-Asn-Glu-Asn-Cys-Gly-Ser-Met-His-
Pro-Asn-Gly-Gly-Leu-Asn-Asp-Ile-Pro-Cys-Pro-Trp-Lys-
Leu-Pro-Phe-Val-Cys-GIu-Val-Glu-Leu.
```

**3.** A double-stranded DNA clone encoding an amino acid sequence for a precursor of lectin-like substance with binding ability to lipopolysaccharides and from <u>Periplaneta</u> <u>americana</u>, which comprises a single-stranded DNA having nucleotides with the sequence of

```
5'--- ATG ATG AAT ACG AGA GCC TTA CTG CCC CTG TCA GTA CTA
       CTC ATG GCT ACC TTG TGT CTG TGT GAG CTG CCT ATC CCC
       ATA CTG CAA AGG TTC GTG CGT TCA GTT CCT GAG GAG TGT
       CCT ATA GCT GAC CCG AGT GAC TTC AAG TTT TCG ATC ACG
       AGC AAT AGG AAC AAG ACT GGC CAC TGG ACG GCG CAG GTG
       CGA CTG GAA CAC GGG GAA CAT GAA CAG TCA GGC TCT AAC
       CAA CAT AAC AGA GAT CTC TGG CAA GTG GAT CTG GAG CAG
       ACC ACA ACC ACT TGT GCA GGC GTC AAG TCT GTT CAG ATC
```

```
ATT ACT ACA ATC ACA GCA CCT CCT CCT ACT GCC GCT CCC

AGT ATT CCT CCA GGA TAC GAG TTG TCC GCT GTT CTG GGA

TAT TAC AAA TTT CAT AAA ACT CCG AAG ACT TGG GAT GAA

GCA AGA ATA ATC TGT CAA CAG GAG GGA GGA CAT CTG GTC

ATA ATA AAT TCG GAG GAC GAA TCT AAA GTT CTT CAA AAC

CTT TTC AGC AAA GTG ACG AAG ACG GAA GGA GCA ACA AAC

AAC GAC TAC ATA TTT ATC GGC ATT CAT GAT AGA TTT GTT

GAA GCA GAA TTC ATC ACG ATA TTT GGA AAA CCT CTG GCT

ACT ACT GGA TTC ACT CGT TGG GTG GAC TCT ATC CAA CCT

GAC AAT GCT GGA GGT AAC GAG AAC TGT GGC TCC ATG CAT

CCT AAT GGC GGT TTG AAC GAT ATA CCA TGC CCG TGG AAA

CTG CCC TTC GTC TGT GAA GTG GAG CTC
```

wherein A, C, G and T are, respectively, a deoxyribonucleotide having adenine, cytosine, guanine or thymine base and the sequence is given as that of each codon encoding a specified amino acid, or a nucleotide sequence with a degeneracy to the sequence, and another single-stranded DNA complementary to the first single-stranded DNA.

4. A plasmid with an insert of a double-stranded DNA clone encoding an amino acid sequence for a precursor of lectin-like substance with binding ability to lipopolysaccharides and from Periplaneta americana, said insert comprising a single-stranded DNA having nucleotides with the sequence of

```
5'--- ATG ATG AAT ACG AGA GCC TTA CTG CCC CTG TCA GTA CTA

       CTC ATG GCT ACC TTG TGT CTG TGT GAG CTG CCT ATC CCC

       ATA CTG CAA AGG TTC GTG CGT TCA GTT CCT GAG GAG TGT

       CCT ATA GCT GAC CCG AGT GAC TTC AAG TTT TCG ATC ACG

       AGC AAT AGG AAC AAG ACT GGC CAC TGG ACG GCG CAG GTG

       CGA CTG GAA CAC GGG GAA CAT GAA CAG TCA GGC TCT AAC

       CAA CAT AAC AGA GAT CTC TGG CAA GTG GAT CTG GAG CAG

       ACC ACA ACC ACT TGT GCA GGC GTC AAG TCT GTT CAG ATC

       ATT ACT ACA ATC ACA GCA CCT CCT CCT ACT GCC GCT CCC
```

13

```
AGT ATT CCT CCA GGA TAC GAG TTG TCC GCT GTT CTG GGA
TAT TAC AAA TTT CAT AAA ACT CCG AAG ACT TGG GAT GAA
GCA AGA ATA ATC TGT CAA CAG GAG GGA GGA CAT CTG GTC
ATA ATA AAT TCG GAG GAC GAA TCT AAA GTT CTT CAA AAC
CTT TTC AGC AAA GTG ACG AAG ACG GAA GGA GCA ACA AAC
AAC GAC TAC ATA TTT ATC GGC ATT CAT GAT AGA TTT GTT
GAA GCA GAA TTC ATC ACG ATA TTT GGA AAA CCT CTG GCT
ACT ACT GGA TTC ACT CGT TGG GTG GAC TCT ATC CAA CCT
GAC AAT GCT GGA GGT AAC GAG AAC TGT GGC TCC ATG CAT
CCT AAT GGC GGT TTG AAC GAT ATA CCA TGC CCG TGG AAA
CTG CCC TTC GTC TGT GAA GTG GAG CTC
```

wherein A, C, G and T are, respectively, a deoxyribonucleotide having adenine, cytosine, guanine or thymine base and the sequence is given as that of each codon encoding a specified amino acid, or a nucleotide sequence with a degeneracy to the sequence, and another single-stranded DNA complementary to said first single-stranded DNA.

**5.** A process for the preparation of a double-stranded DNA clone which encodes a precursor of lectin-like substance with binding ability to lipopolysaccharides and from Periplaneta americana, the process comprising steps of converting mRNA from fat-body of Periplaneta americana into cDNA, constructing a cDNA library with use of the cDNA and vector/primer DNA to carry out a transformation of Escherichia coli, previously synthesizing a mixture of 24 tetracosamers, each consisting of 20 deoxyribonucleotides of the formula

```
5' --- GGA  TCIGCA  ATIGGA  CAT  TC
         G        G       G    C              (A)
                  T
```

wherein I is inosine residue and was inserted in each position where anyone of A, C G and T may occupy,
which is complementary to mRNA corresponding to
    Glu-Cys-Pro-Ile-Ala-Asp-Pro
of 5th to 11th amino acids from 5'-terminal in an amino acid sequence for lectin-like substance and from a body fluid of Periplaneta americana, labeling at 5'-terminal of each of said synthesized oligodeoxyribonucleotides, and screening said transformants through a hybridization using the labeled oligodeoxyribonucleotides as probes to obtain a positive clone which hybridize with these probes.

14

# FIG. 1

RP: Region of protein with binding ability to lipopolysachharides

# FIG. 2 (⅓)

```
TTCGTTGGGC AACACGAAGA CAAGCTTTAA GTCATA ATG ATG AAT ACG AGA GCC        54
                                          Met Met Asn Thr Arg Ala
                                                      -30

TTA CTG CCC CTG TCA GTA CTA CTC ATG GCT ACC TTG TGT CTG TGT GAG        102
Leu Leu Pro Leu Ser Val Leu Leu Met Ala Thr Leu Cys Leu Cys Glu
        -25                 -20                 -15

CTG CCT ATC CCC ATA CTG CAA AGG TTC GTG CGT TCA GTT CCT GAG GAG        150
Leu Pro Ile Pro Ile Leu Gln Arg Phe Val Arg Ser Val Pro Glu Glu
        -10                 -5              -1   1               5

TGT CCT ATA GCT GAC CCG AGT GAC TTC AAG TTT TCG ATC ACG AGC AAT       198
Cys Pro Ile Ala Asp Pro Ser Asp Phe Lys Phe Ser Ile Thr Ser Asn
                10                  15                  20

AGG AAC AAG ACT GGC CAC TGG ACG GCG CAG GTG CGA CTG GAA CAC GGG        246
Arg Asn Lys Thr Gly His Trp Thr Ala Gln Val Arg Leu Glu His Gly
            25                  30                  35

GAA CAT GAA CAG TCA GGC TCT AAC CAA CAT AAC AGA GAT CTC TGG CAA        294
Glu His Glu Gln Ser Gly Ser Asn Gln His Asn Arg Asp Leu Trp Gln
            40                  45                  50

GTG GAT CTG GAG CAG ACC ACA ACC ACT TGT GCA GGC GTC AAG TCT GTT       342
Val Asp Leu Glu Gln Thr Thr Thr Thr Cys Ala Gly Val Lys Ser Val
            55                  60                  65

CAG ATC ATT ACT ACA ATC ACA GCA CCT CCT CCT ACT GCC GCT CCC AGT      390
Gln Ile Ile Thr Thr Ile Thr Ala Pro Pro Pro Thr Ala Ala Pro Ser
70                  75                  80                  85

ATT CCT CCA GGA TAC GAG TTG TCC GCT GTT CTG GGA TAT TAC AAA TTT        438
Ile Pro Pro Gly Tyr Glu Leu Ser Ala Val Leu Gly Tyr Tyr Lys Phe
                90                  95                  100

CAT AAA ACT CCG AAG ACT TGG GAT GAA GCA AGA ATA ATC TGT CAA CAG       486
His Lys Thr Pro Lys Thr Trp Asp Glu Ala Arg Ile Ile Cys Gln Gln
            105                 110                 115

GAG GGA GGA CAT CTG GTC ATA ATA AAT TCG GAG GAC GAA TCT AAA GTT      534
Glu Gly Gly His Leu Val Ile Ile Asn Ser Glu Asp Glu Ser Lys Val
            120                 125                 130
```

# FIG. 2 (2/3)

```
CTT CAA AAC CTT TTC AGC AAA GTG ACG AAG ACG GAA GGA GCA ACA AAC   582
Leu Gln Asn Leu Phe Ser Lys Val Thr Lys Thr Glu Gly Ala Thr Asn
        135                 140                 145

AAC GAC TAC ATA TTT ATC GGC ATT CAT GAT AGA TTT GTT GAA GGA GAA   630
Asn Asp Tyr Ile Phe Ile Gly Ile His Asp Arg Phe Val Glu Gly Glu
150                 155                 160                 165

TTC ATC ACG ATA TTT GGA AAA CCT CTG GCT ACT ACT GGA TTC ACT CGT   678
Phe Ile Thr Ile Phe Gly Lys Pro Leu Ala Thr Thr Gly Phe Thr Arg
                170                 175                 180

TGG GTG GAC TCT ATC CAA CCT GAC AAT GCT GGA GGT AAC GAG AAC TGT   726
Trp Val Asp Ser Ile Gln Pro Asp Asn Ala Gly Gly Asn Glu Asn Cys
            185                 190                 195

GGC TCC ATG CAT CCT AAT GGC GGT TTG AAC GAT ATA CCA TGC CCG TGG   774
Gly Ser Met His Pro Asn Gly Gly Leu Asn Asp Ile Pro Cys Pro Trp
            200                 205                 210

AAA CTG CCC TTC GTC TGT GAA GTG GAG CTC TGA CAACAGACGG TTCCGCACAT   827
Lys Leu Pro Phe Val Cys Glu Val Glu Leu ###
        215                 220         223

CTCAAAGATT GTAACTTTGA GACAGTCTTA AGTCCAATAT TGTAATTAAA ACTTGCATTT   887

GTAAGTTATT TATGATAATG TTATTGTTAG TGAAAGATAT CTGCCATTAT AAATAACAAA   947

ATATTGTTCC AAAGAAATTT TTAAAATTTA TTTTACGACG CTTTATCAAC TGCGATACCA  1007

ATCTAGCGTC TCAGTGAGAT GAAGGTGACT ATTACACTCT TACTACGTCA TGCTACTTTT  1067

GACCAATAAA ACGGTACGAA AGGACGTATT TCAACCAATC ATGGCTGCTT ATCGCACAAT  1127

TTTATCGCGT CCCTAGCATT TGTTTAATTT TATCGCGTCC CTAGCATTTG TTTAATTTTA  1187

TCGCGTCCCT AGCATTTGTT TAATTTTATC GCGTCCCTAG CATTTGTTTA ATTTTATCGC  1247

GTCCCTAGCA TTTGTTTAAT TTTATCGCGT CCCTAGCATT TGTTTAATTT TATCGCGTCC  1307

CTAGCATTTG TTTAATTTTA TCGCGTCCCT AGCATTTGTT TAATTTTATC GCGTCCCTAG  1367

CATTTGTTTA ATTTTATCGC GTCCCTAGCA TTTGTTTAAT TTTATCGCGT CCCTAGCATT  1427
```

17

# FIG. 2 (3/3)

```
TGTTTAATTT TATCGCGTCC CTAGCATTTG TTTAATTTTA TCGCGTCCCT AGCATTTGTT 1487

TAATTTTATC GCGTCCCTAG CATTTGTTTA ATTTTATCGC GTCCCTAGCA TTTGTTTCTT 1547

TGTTTGCCAA CATTTGAAAC TGCGCTGGTC TGGACCTCAA AAAGAAAATA TATATATATA 1607

TATATATTTA TATATATAAA ATTACAAACC ACTCCAGTTG ATGCACAGCA GTTTCAAATA 1667

TGACTCTCAT TGGCATTCAA GAACAAGAAT TAATAAAAGT CATTGATCAT ACCTATGCAT 1727

CTTCTGAAAT CCGATTTACA AATAAATGAA GAGCACCATT CGGAAATCCT GAATAAGTTG 1787

AATACACCAT GTAGGCCTAA ATCAACGAGT TCCACTTCTA TTACGCACAC GTCCAATATA 1847

ACATCAATTG AACCACCAAC CACATTCAAA TTTGAAAATT GTACATTCAA TAATTATTCA 1907

TTTTAAAATT ATTCATTCGG AAATTCTGAA TAAGTTGAAT ACACCATGTA GGCCTAAATC 1967

AACGAGTTCC ACTTCTATTA CGCACACGTC CAATATAACA TCAATTGAAC CACCAACCAC 2027

ATTCAAATTT GAAAATTGTA CATTAAATAA TTATTCCTTT TAAAATTATT CATGTTTATT 2087

TTTTATGTCA TCGTCGTTAA TTAACACTTT TCTAACACTT GTGTACAGTA TATTAGTTAG 2147

GTTATGTTAT AGCTTCTGCC ATATGATATT ATGGATAGTC ACGTATCAGA GATTGTTTAA 2207

TATTAAGATT TATTGAAAAT CATCTGTCAA GTGACGTTGA TTACTGGGAT TCGGATAATT 2267

GAAGTGGAAT GTTGCATTTT AATAAAAATG AAACTGAATC                        2307
```

# FIG. 3

RP: Region of protein with binding ability
to lipopolysachharides